(19) **Europäisches Patentamt**
**European Patent Office**
**Office européen des brevets**

(11) **EP 2 243 031 B1**

(12) **EUROPEAN PATENT SPECIFICATION**

(45) Date of publication and mention
of the grant of the patent:
**30.05.2012 Bulletin 2012/22**

(51) Int Cl.:
***G01N 33/68*** (2006.01)

(21) Application number: **09703185.0**

(86) International application number:
**PCT/US2009/031433**

(22) Date of filing: **20.01.2009**

(87) International publication number:
**WO 2009/094331 (30.07.2009 Gazette 2009/31)**

(54) **METHODS OF PREDICTING ANTIBODY SOLUBILITY**

VERFAHREN ZUR VORHERSAGE VON ANTIKÖRPERLÖSLICHKEIT

PROCÉDÉS DE PRÉDICTION DE SOLUBILITÉ D'ANTICORPS

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **21.01.2008 US 22385 P**

(43) Date of publication of application:
**27.10.2010 Bulletin 2010/43**

(73) Proprietor: **Janssen Biotech, Inc.
Horsham, PA 19044 (US)**

(72) Inventors:
• **O'NEIL, Karen
Radnor, PA 19087 (US)**
• **JACOBS, Steven
Radnor, PA 19087 (US)**
• **WU, Sheng-Jiun
Radnor , PA 19087 (US)**
• **BETHEA, Deirdra
Radnor, PA 19087 (US)**
• **FENG, Yiqing
Radnor, PA 19087 (US)**

(74) Representative: **Goodfellow, Hugh Robin
Carpmaels & Ransford
One Southampton Row
London
WC1B 5HA (GB)**

(56) References cited:
**WO-A2-2006/131013     US-A- 6 153 438
US-A1- 2004 067 577**

• **RUPPERT S ET AL: "Correlation between the
osmotic second virial coefficient and the
solubility of proteins", January 2001 (2001-01),
BIOTECHNOLOGY PROGRESS, VOL. 17, NR. 1,
PAGE(S) 182-187, XP002612961, ISSN: 8756-7938
* abstract ***
• **TESSIER PETER M ET AL: "Direct measurement
of protein osmotic second virial cross
coefficients by cross-interaction
chromatography", PROTEIN SCIENCE, vol. 13,
no. 5, May 2004 (2004-05), pages 1379-1390,
XP002612962, ISSN: 0961-8368**
• **TESSIER P M ET AL: "Rapid measurement of
protein osmotic second virial coefficients by self-
interaction chromatography", BIOPHYSICAL
JOURNAL BIOPHYS. SOC USA, vol. 82, no. 3,
March 2002 (2002-03), pages 1620-1631,
XP002612963, ISSN: 0006-3495**
• **PATRO ET AL.: 'Self-Interaction
Chromatography: A Tool for the Study of Protein-
Protein Interactions in Bioprocessing
Environments.' BIOTECHNOLOGY AND
BIOENGINEERING vol. 52, 1996, pages 193 - 203**
• **GAGNON ET AL.: 'An adaptation of hydrophobic
interaction chromatography for estimation of
protein solubility optima.' JOURNAL OF
PHARMACEUTICAL AND BIOMEDICAL
ANALYSIS vol. 16, 1997, pages 587 - 592**

**Description**

**Field of the Invention**

The invention relates to methods of characterizing candidate therapeutic antibodies using information for the potential for self-interaction indicative of limited solubility using a heterologous antibody in a chromatographic assay measuring interaction.

**Description of the Related Art**

Many therapeutic antibody candidates now in the clinic were selected and optimized from large libraries of antibody variants. Typically, early stage screens for selecting among candidate antibodies include binding affinity and specificity for target antigens, and in vitro bioactivity assays. Frequently, at the early selection stage, only small amounts of an antibody candidate are available for characterization.

Once a mAb candidate is selected for development, there is a significant time investment required for scale-up production and purification before the candidate proceeds to the stages of development and formulation. Thus, large amounts of protein are often not available for biophysical characterization until several years have been invested in an antibody candidate. A currently employed method of therapeutic Mab administration is by the subcutaneous deposit, and generally requires that the therapeutic molecule be formulated at a concentration which may exceed 100 mg/mL. Thus, a method for early assessment of the potential, e.g., formulation at high concentration or solubility under physiologically relevant conditions, which can be performed on small quantity preparations of candidate therapeutic monoclonal antibodies would be valuable in selecting among variants to take forward in the development process.

A method for determining the biophysical properties which afford the potential for high concentration formulation would be valuable for improving the probability of a antibody being successfully advanced as a therapeutic development candidate. Preferably, the method would use the amounts of test material available prior to clonal scale-up for expression which amount may be less than 1 milligram of purified recombinant protein.

**SUMMARY OF THE INVENTION**

The invention relates to a method for screening among antibodies with binding specificity for a target antigen those antibodies with desirable biophysical properties, such as solubility in aqueous medium and reduced susceptibility to aggregation. The method of the invention provides a chromatographic method for determining antibody solubility by measuring interactions between a first antibody and a second antibody, the method comprising:

a) flowing a solution comprising a substantially homogeneous solution of the first antibody over a solid support contained in a defined column volume, wherein the second antibody is immobilized on a surface of the solid support; and

b) measuring the time (retention time, $t_r$) required for an amount of the solution comprising the first antibody to exit said column volume, wherein the retention time ($t_r$) is a measure of interaction between the first antibody and the second antibody which correlates with the solubility of the first antibody in a pharmaceutically acceptable formulation, wherein the first and second antibody are different antibodies.

In one aspect of the invention, the subject antibody is a therapeutic antibody candidate with binding specificity for a target antigen antibody and the subject antibody is a sequence altered variant of the second antibody. In another aspect of the invention, the first antibody is a therapeutic antibody candidate and is a member of a group of variants derived from a parental antibody and the second antibody is the parental antibody. In yet another aspect of the invention, the second antibody is a heterogenous preparation of antibody molecules. In a specific embodiment, the second antibody is polyclonal IgG.

In one embodiment of the invention, the method of the invention is used to rank the biophysical suitability of an antibody for further development. In one aspect of the method of the invention the biophysical property of the antibody is the ability of the antibody to be concentrated without aggregation in aqueous medium. In a particular embodiment, the method of the invention is used to select an antibody which is soluble in aqueous medium at a concentration of 100 mg per ml. In another example of the method of the invention, the method is used to rank the biophysical properties of a collection of antibody candidates having binding specificity for a target antigen. In a particular example, the method of the invention is used as a screen and categorize multiple antibody candidates as having more desirable or less desirable biophysical properties.

## BRIEF DESCRIPTION OF THE SEVERAL VIEWS OF THE DRAWING

FIG. 1    is a plot of the B22 value versus solubility in various formulations for a single human monoclonal antibody: 1) 10 mM histidine pH 5.5, 280 mM sucrose 2) 10 mM sodium citrate pH 5.5, 280 mM sucrose 3) 10 mM sodium acetate pH 5.5, 280 mM sucrose 4) 10 mM sodium acetate pH 5.0, 280 mM sucrose, 30 mM glycine, 30 mM mannitol 5) 10 mM sodium acetate pH 5.0, 280 mM sucrose, 30 mM glycine, 30 mM mannitol, 150 mM sodium chloride.

FIG. 2    shows chromatographic traces of test proteins analyzed on MAB67-coupled chromatographic material by HPLC.

FIG. 3    shows a plot of the values for $B_{22}$ measured by SLS in PBS and k' values measured in PBS using a column of MAB67-coupled chromatographic material by HPLC for homogenous solutions of antibodies, SYNAGIS (pavilizumab) or Mab1, or an antibody-derived dimeric fusion protein ENBREL (enteracept) used to derive a coefficient relating $B_{22}$ to k' for such proteins.

FIG. 4    shows chromatographic traces for MAB67 and variants Mut2, Mut3, Mut4, Mut5, and Mut6 on MAB67-coupled chromatographic material by HPLC.

FIG. 5    shows chromatographic traces of MAB67, Mut6, pavilizumab, and enteracept on a hIgG-coupled chromatography column in PBS

FIG. 6    shows chromatographic traces comparing the NHS-activated and tresyl-group resins on the same LC system and where both columns were coupled with hIgG at a coupling density of 30mg/ml, used 20 ul injection volume, and 0.1ml/min flow rate: the results in A are from the NHS-activated resin and from B, the Tosoh resin.

FIG. 7    shows chromatographic traces of elution profiles NHS-activated resin coupled to hIgG at 30mg/ml coupling density and 20ul injection volume: MAB 15 = bold line and MAB16 = thin line.

FIG. 8    shows chromatographic traces of elution profiles of three related Mabs, on a column containing NHS-activated resin coupled to hIgG at 30mg/ml coupling density and a 0.1ml/min flow rate; A 10 ul injected and B, 20ul injected..

FIG. 9    shows chromatographic traces of the elution profile for MAB453 on a NHS-activated resin coupled to hIgG at 10mg/ml, 20mg/ml, and 30mg/ml, respectively.

FIG. 10    shows chromatographic traces of antibody candidates derived from parent molecule 5645 on a hIgG-coupled chromatography column in PBS.

FIG. 11    shows chromatographic traces of humanized antibodies derived from a murine parent molecule, P86, on a hIgG-coupled chromatography column in PBS.

## DETAILED DESCRIPTION OF THE INVENTION

### Abbreviations

AIC = antibody interaction chromatography; $B_{22}$ = osmotic second virial coefficient; B23 = osmotic second virial cross coefficient; IgG =antibodies, polyclonal or monoclonal; CDR = complementarity-determining regions; CIC = cross-interaction chromatography; Ig = immunoglobulin; Mab = monoclonal antibody; SIC = self interaction chromatography; SLC = static light scattering; PBS = phosphate buffered saline.

Definitions

In the present invention, the term "antibody", "monoclonal antibody" or "Mab" encompasses all classes and subclasses of antibody of human or nonhuman origin along with variants, derivatives, fragments, and fusion proteins thereof, which may be suitable for use in prevention, treatment, or management of diseases of humans or animals. The term "antibody" herein is used in the broadest sense. As used herein, an "antibody" includes whole antibodies and any antigen binding fragment or a single chain thereof. Thus, the antibody includes any polypeptide containing molecule that comprises at least a portion of an immunoglobulin molecule, such as but not limited to at least one heavy or light chain or a ligand binding portion thereof, a heavy chain or light chain variable region, or complementarity-determining region (CDR) of a heavy or light chain variable region, a framework (FR) region, or any portion thereof, or at least one portion of a binding protein, which can be incorporated into an antibody of the present invention. The term "antibody" is further intended to encompass antibodies, digestion fragments, specified portions and variants thereof, including antibody mimetics or comprising portions of antibodies that mimic the structure and/or function of an antibody or specified fragment or portion thereof, including single chain antibodies and fragments thereof. Functional fragments include antigen-binding fragments to a preselected target. Examples of binding fragments encompassed within the term "antigen binding portion" of an antibody include (i) a Fab fragment, a monovalent fragment consisting of the VL, VH, CL and CH, domains; (ii) a F(ab')2 fragment, a bivalent fragment comprising two Fab fragments linked by a disulfide bridge at the hinge region; (iii) a Fd fragment consisting of a VH and a CH domain; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Each VH and VL is composed of three CDRs and four FRs, arranged from amino-terminus to carboxy-terminus in the following order: FR1, CDR1, FR2, CDR2, FR3, CDR3, FR4. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al, 1988 Science 242:423-426, and Huston et al. 1988 Proc. Natl. Acad Sci. USA 85:5879-5883 or structures with less flexabilty between the VL and VH domains which themselves dimerize or multimerize (diabodies and triabodies). Such single chain antibodies are also intended to be encompassed within the term "antigen-binding portion" of an antibody. Antibody fragments are obtained using conventional techniques known to those with skill in the art, and the fragments are screened for binding specificiy and affinity and bioactiviy in the same manner as are intact antibodies.

By "antibody having specificity for a target" is meant that the antibody or a preparation containing the antibody demonstrates specific binding to a known antigenic target or portion or epitope thereof. As used herein, "specific binding" refers to antibody binding to a predetermined antigen. Typically, the antibody binds with a dissociation constant ($K_D$) of $10^{-7}$ M or less, and binds to the predetermined antigen with a $K_D$ that is less than its $K_D$ for binding to a non-specific antigen (e.g., BSA, casein, or any other specified polypeptide) whether the $K_D$ for binding to proteins other than the predetermined antigen are known or unknown.

By "physiologically acceptable formulation" or "physiologically acceptable aqueous solution" is meant solutions suitable for use in the body, most often in the blood, and which must be able to mix freely with blood without unacceptably compromising its components, such as creating precipitates which significantly block flow in small vessels, destroying an unacceptable portion of its formed elements (cells, platelets), introducing agents or creating water, ionic or molecular imbalances destructive to body cells and tissues, or causing harmful physiologic activities such as inappropriate acceleration or inhibition of heartbeat, nerve conduction or muscle contraction, and the like.

By "recombinant antibody" is meant an antibody of defmed sequence produced by recombinant methods. By "recombinant antibody" is meant all antibodies that are prepared, expressed, created or isolated by recombinant means, such as antibodies expressed using a recombinant expression vector transfected into a host cell, antibodies isolated from, e.g., a recombinant, combinatorial human antibody library (described further below), antibodies isolated from an animal (e.g., a mouse) or antibodies prepared, expressed, created or isolated by any other means that involves splicing of human or other immunoglobulin gene sequences to other DNA sequences. Such recombinant antibodies may have variable and constant regions derived from human germline immunoglobulin sequences. In certain embodiments, however, such recombinant antibodies are subjected to directed to random in vitro mutagenesis producing new variants which are isolatable and of defined sequence.

An "isolated antibody", as used herein, is intended to refer to an antibody that is substantially free of other antibodies having different antigenic specificities.

By "osmotic second virial coefficient", "second virial coefficient" or "B22" is meant a measure of the magnitude and direction of the deviations of a non-ideal solution from ideality with respect to the magnitude and direction of self-interaction between two molecules having identical chemical compositions. Two experimental methods have been employed to measure $B_{22}$ values for recombinant proteins: static light scattering (Demoruelle, et al., Acta Crystallogr D Biol Crystallogr 58: 1544-8, 2002; George & Wilson, Acta Crystallogr D Biol Crystallogr 50: 361-5, 1994; Guo, et al., Journal of Crystal Growth 196: 424-433, 1999) and self-interaction chromatography or "SIC" (Garcia, et al., Biotechnol Prog 19: 575-9, 2003; Tessier, et al., Acta Crystallogr D 58: 1531-5, 2002a; Tessier, et al., Biophys J 82: 1620-31, 2002b).

By "osmotic second virial cross coefficient "or "B23" is meant a measure of the magnitude and direction of the deviations of a non-ideal solution from ideality with respect to the magnitude and direction of the interactions between two molecules in a mixture having non-identical chemical compositions. Methods that have been used to characterize protein cross-association are membrane osmometry (McCarty and Adams 1987; Moon et al. 2000), light scattering (Steiner 1953a), sedimentation equilibrium (Howlett and Nichol 1973; Clarke and Howlett 1979), size-exclusion chromatography (Nichol and Winzor 1964; Nichol et al. 1967; Gilbert and Kellett 1971), fluorescence depolarization (Steiner 1953b), and turbidity methods (Howell et al. 1995). A method of determining $B_{23}$ by immobilized protein-interaction chromatography has been described (Tessier et al. Protein Sci 13:1379-1390, 2004) where different homogenous proteins are studied and one is immobilized while the other is in the solvent phase and termed cross-interaction chromatography (CIC).

By $k'$ is meant the chromatographic retention factor derived from the difference in either the retention time ($t_r$) or retention volume ($V_r$), respectively, for a subject antibody during column chromatography on an antibody-derivatized material and the time ($t_m$) or volume ($V_o$) where no interaction takes place on the same column.

$$k' = t_r - t_m / t_m \quad \text{OR} \quad k' = V_r - V_o / V_o$$

Where $t_r$ and $V_r$ are the retention time or volume of the sample on the protein derivatized column, respectively, $t_m$ and Vo are the retention time or volume of an unretained sample respectively, determined by injecting protein samples on a control column in which no protein was immobilized (underivatized). The retention time of acetone on both columns is used to standardize column volumes using the following equation:

$$t_m = \frac{t_{pc}}{t_{ac}} \cdot t_{al}$$

where $t_{pc}$ is the retention time of the protein on the control column, $t_{ac}$ the retention time of acetone on the underivatized column, and $t_{al}$ the retention time of acetone on the antibody-coupled column. Vo can be derived from the following equation

$$V_o = \frac{V_{pc}}{V_{ac}} V_{al}$$

where $V_{pc}$ is the retention volume of the protein on the underivatized column, $V_{ac}$ the retention volume of acetone on the control column, and $V_{al}$ the retention volume of acetone on the antibody-coupled column.

By "antibody interaction chromatography" or "AIC" is meant that an antibody related in class and structure is affixed to a solid support material and used as the stationary phase as fluid containing a test protein of the same class and structure in a solution phase is passed over the material. A non-limiting example of AIC is where a preparation of human IgG molecules is affixed to a solid support and a homogeneous solution of test human IgG antibody is flowed over the support in order to measure the level of interaction of the test antibody with the heterologous antibodies on the support.

By "parental antibody" is meant an antibody that binds a specific target antigen isolated from a primary screen of a phage display library or an antibody isolated from an animal after immunization with a specific target antigen possibly followed by humanization of the sequences wherein the parent antibody primary amino acid sequences is modified by making amino acid substitutions with the intention of improving, enhancing, or modifying one or more biophysical or biological activities of the antibody.

By "protein variant" is meant an antibody derived from a parental antibody by mutation of at least one amino acid in order to change the biological or biophysical properties of said parental antibody. Non-limiting examples of properties to be altered include solubility, thermal stability, affinity for target antigen, humanization, and addition or removal of potential glycosylation sites.

## Description the Invention

The osmotic second virial coefficient ($B_{22}$) provides a functional measure of the solubility characteristics of a protein insofar as the coefficient can be derived by at least two types of protein self-interaction phenomenon: static light scattering measurements of dilute homogeneous protein solutions and tendency to exhibit retarded flow as measured by self-interaction chromatography (SIC). Static light scattering is reliable; however the measurements are time consuming, require large amounts of recombinant protein and the use of specialized equipment. In contrast, SIC is a simpler method for determining $B_{22}$ values, however, still requires relatively large amounts of a single protein to immobilize on the column material. For SIC measurements, the protein of interest is attached to an immobilized chromatography support and using conventional chromatography equipment the identical protein is flowed over the column in an appropriate buffer. The $B_{22}$ value can then be calculated from the chromatographic retention factor, $k'$, using the observed retention parameters. Proteins with more negative $B_{22}$ values are retained longer on the column due to their attraction to the stationary phase. Proteins that are more soluble in the mobile phase buffer pass rapidly through the column and have more positive $B_{22}$ values.

$B_{22}$ is related to thermodynamic properties contributed by hydrophobic effects, van der Waals interactions, hydration forces and electrostatic interactions to provide a measure of the aggregation properties of the molecule (Demoruelle, et al., Acta Crystallogr D Biol Crystallogr 58: 1544-8, 2002). The $B_{22}$ value reflects both the magnitude and sign of the self-interaction between two protein molecules in solution. Briefly, a positive $B_{22}$ value indicates repulsive interactions between protein molecules while negative values represent attractive interactions (Neal, et al., Journal of Crystal Growth 196: 377-387, 1999). Protein solutions with very negative $B_{22}$ values indicate a tendency for aggregation and precipitation. Peptides (approximately 4000 Da) have also demonstrated the correlation between solubility and $B_{22}$ values (Valente, et al., Curr Pharm Biotechnol 6: 427-36, 2005). One utility of $B_{22}$ is as a method for identifying conditions for protein crystallization (the crystallization window), see WO05/080422. Crystal growth requires an ideal set of solution conditions where ordered protein self-interaction is promoted and non-specific aggregation is minimized. In contrast, for therapeutic applications it is necessary to identify the protein variant that is most soluble in a pharmaceutically relevant buffer.

WO2006/131013 discloses SIC of antibody-derived molecules.

Human protein solubility has been studied generally using a variety of methods (Bondos, et al. 2006 Current Analytical Chemistry, 2(2):157-170). The use of $B_{22}$ to gauge protein solubility has been studied for monomeric, globular proteins such as lysozyme and bovine alpha-chymotrypsinogen A (Neal, et al., *supra*). Applicants have unexpectedly found that a measurement analogous to SIC for measuring $B_{22}$ values is useful for predicting the solubility of homogeneous solutions of monoclonal antibodies and, therefore, for selecting among a myriad of therapeutic antibody candidates with the most favorable biophysical properties for development.

In the method of the invention, protein-protein interactions are used to measure parameters predictive of the susceptibility of a protein to aggregate and precipitate from an aqueous solution, preferable a biocompatible or pharmaceutical acceptable solution. Mathematically, the relationship between solubility and interaction has been described in terms of the measurements that range of interaction (linear dimension) between molecules (Haas et al. 1999 J Phys Chem B. 103:2808-2811). These considerations find application to the problem of determining solubility of a specific protein, and in the present example, IgG or IgG-derived constructs which are candidates for therapeutic protein development. Unlike SIC, where the test protein in the mobile phase is identical to the immobilized protein, applicants have demonstrated that antibody interactions with a variant antibody, or in other cases antibody interactions with heterologous mixtures of antibodies can be used to measure parameters predictive of the susceptibility of the antibody to aggregation when brought into a homogenous solution of purified antibody. The present discovery allows rapid screening of a collection of antibody candidate molecules for attributes related to the ability to be concentrated during formulation, such as solubility and susceptibility to self-aggregate.

In one aspect of the invention, the interaction between a Mab candidate and a test variant which is a variant of a parental Mab can be used to measure parameters predictive of the susceptibility of the antibody to aggregation. In one aspect of the invention, the interaction between a Mab candidate and polyclonal IgG can be used to measure parameters predictive of the susceptibility of the antibody to aggregation.

In one aspect of the invention, the interaction between a mAb candidate and a test variant which is a variant of a parental Mab are measured by chromatographic methods to yield one or more retention parameters, such as but not limited to $t_r$ or $V_r$, which parameters are predictive of the solubility of the protein or susceptibility of the protein to aggregate. In one aspect of the invention, the interaction between a mAb candidate and polyclonal IgG can be used to measure parameters predictive of the susceptibility of the antibody to aggregate.

Whereas B22 and B23 measurements by chromatography methods, SIC and CIC, respectively, have been shown to be reflective of protein interaction in solution, the methods of the invention use an antibody-immobilized column in a chromatographic retention measurement to predict Mab solubility and, in particular, the potential for high concentration formulation.

The immobilized protein in the method of the invention is a non-identical but structurally related protein, which is either homogeneous or heterogeneous. An example of a structurally related protein used in the method of the invention is a parent Mab, from which variant Mabs made by amino acid substitution have been created. In an example of a heterogeneous protein interaction chromatography method of the invention, the parent Mab may be immobilized by covalent linkage to the solid phase and solutions of the variant Mabs flowed through the column and retention time ($t_r$) measured. In another embodiment, a variant Mab may be immobilized and the same or different structurally related Mab may be flowed over the column and $t_r$ measured. In yet another embodiment, a preparation containing a mixture of antibodies, such as a polyclonal antibody, or heterologous antibodies of undetermined specificity may be immobilized and used in the $t_r$ measurement of an antibody or selection or family of antibodies. In all these embodiments, elution volume ($V_r$) may be measure instead of retention time as defined above. The $t_r$ or $V_r$ measurements are then used to calculate the retention factor (k') related to each of the antibodies in the mobile phase for a particular column and solution conditions.

Parameters used for prediction of aggregation in the method of invention include increased retention time or retention volume, increased peak width, presence of multiple peaks, or lack of detectable peaks of of a subject antibody on an antibody-coupled support in a column with respect to a soluble reference protein assayed on the same column. Comparison of the retention times or volumes between two antibodies on a particular column can be used to rank the susceptibility of these antibodies to aggregation where the antibody with the longer retention time (or larger retention volume) is predicted to be more prone to aggregation. In another embodiment, the chromatographic peak width is used to rank the susceptibility of aggregation wherein antibodies having a wider peak width are more prone to aggregation. In yet another embodiment, a series of antibodies is ranked for susceptibility to aggregation using these parameters. Retention time or volume can also be used to estimate the solubility of an antibody in the chromatographic mobile phase buffer provided the retention time of this antibody is compared a reference antibody or antibodies with known solubility in such a buffer.

**Antibodies and Variants**

Monoclonal antibodies (Mabs) have been fully realized as an important therapeutic class of molecules. Owing to their inherent lack of toxicity and high degree of specificity and affinity for their targets, monoclonal antibody therapeutics, especially human or humanized versions of antibodies have proved efficacious for long-term management of chronic and life-threatening diseases such as Crohn's disease, rheumatoid arthritis, and various types of cancer. As Mabs made by recombinant protein techniques are of defined amino acid sequence and can be cloned into a suitable and stable host cell line, methods for manufacture of Mabs and their approval by regulatory agencies has become widely accepted. More than 100 Mabs and Mab-derived molecules are now in use or in development for treatment of subjects suffering from or at risk for various pathologies.

The classical hybridoma method of obtaining a single antibody species by selecting immortalizing a B-cell from an immunized mouse by fusion with a myeloma cell line was described by Kohler and Milstein in 1975 (Nature 256:495). Recombinant techniques have been applied to the murine sequences in successively more refined engineering to provide more human or human-like antibody characteristics to decrease potential human anti-mouse (HAMA) responses. Such antibodies produced by these techniques are known as "humanized" Mabs.

The construction of libraries of recombinant antibody fragments that are displayed on the surface of filamentous phage, and the selection of phage antibodies against target antigens, have become an important technological tool in generating new monoclonal antibodies for research and clinical applications. Libraries can be constructed from naïve sequences or from B-cells from immunized animals or donors. By incorporating human germline sequences as the framework and constant regions, antibodies obtained by this method are essentially human antibodies and expected to be non-immunogenic when used in human patients as compared to murine Mabs selected from hybridomas (Tomlinson IM: Immunoglobulin genes. In Encyclopedia of Immunology edn 2. Edited by Delves PJ, Roitt I. London: Academic Press; 1998). In one embodiment, the human antibody is selected from a phage library, where that phage library expresses human antibodies (Vaughan et lo al. Nature Biotechnology 14:309-314 (1996): Sheets et al. PITAS (USA) 95:6157-6162 (1998)); Hoogenboom and Winter, J. Mol. Biol., 227:381 (1991); Marks et al. J. Mol. Biol., 222:581 (1991)). Thus, naïve or semisynthetic human antibody libraries can be used in the search for specific antibodies without immunization with respective antigens.

The use of antibody libraries can be further used to improve the affinity of selected antibodies. The construction of mutant antibody libraries based on a parental antibody sequence provides variants which may have greater affinity (or a smaller $K_D$) than the parental antibody. The later process is known as "affinity maturation" of a parental antibody. Similarly, mutant antibody libraries of variants with altered sequences that affect the biophysical properties of the parental antibody can also be constructed. As is known to those skilled in the art, libraries can also be made with the use of appropriate vectors for generation of degenerate sequences, based on the sequences of Mabs derived from hybridoma or humanized versions of murine or other hybridoma derived Mabs.

During the past decade several display methods and other library screening techniques have been developed for isolating monoclonal antibodies (mAbs) from large collections of recombinant antibody fragments. These technologies are now widely exploited to build human antibodies with high affinity and specificity. Using specifically designed libraries, such as directed evolution of binding sites with ultra-high affinity, and innovative selection criteria to identify mAb leads, virtually any specificity and ultra-high affinity (picomolar) Mabs have been discovered. Automation of the technology is making it possible to identify hundreds of different antibody leads, or antibody binding fragments, to a specific therapeutic target. Adalimumab (HUMIRA, a human IgG1 specific for human tumor necrosis factor), was found using such techniques as described in EP0929578. Another technique is epitope imprinting, or guided selection, described in Hoogenboom et al., PCT Publication No. WO 93/06213. The antibody libraries used in this method are preferably scFv libraries prepared and screened as described in McCafferty et al, PCT Publication No. WO 92/01047, McCafferty et al., Nature (1990) 348: 552-554; and Griffiths et al., (1993) EMBO J. 12:725-734. Other recombinant antibodies may be derived from various types of display libraries, such as but not limited to, ribosome display and yeast display, are expected to provide a for the discovery of antibody-based biopharmaceuticals, diagnostics and research reagents increasingly in the future. Other directed evolution platforms recently developed for antibody fragments include retroviral display (Urban, J.H. et al. 2005. Nucleic Acids Res. 33, e35), display based on protein-DNA linkage (Odegrip, R et al. 2004, Proc. Natl. Acad. Sci. USA 101, 2806-2810; Reiersen, H. et al. 2005. Nucleic Acids Res. 33, e10), microbead display by in vitro compartmentalization (Sepp, A., Tawfik, D.S. & Griffiths, A.D. 2002. FEBS Lett. 532, 455-458), in vivo-based growth selection based on the protein fragment complementation assay (PCA) (Mossner, E., Koch, H. & Pluckthun, A. 2001. J. Mol. Biol. 308, 115-122).

One technique which can be used to produce a specific library of antibodies altered variable domains (VH and VL) based upon a parent antibody is by using partially degenerated oligonucleotides (Glaser SM, Yelton DE, Huse WD. 1992. J Immuno 149:3903-3913) while a second technique is by the error-prone PCR method. A third technique is parsimonious mutagenesis where a computer-assisted method for oligodeoxyribonucleotide-directed scanning mutagenesis is used, simultaneously searching all three CDRs of a variable region gene for improved variants in a library to 'probe' the surface of the antigen one or a few aa residues at a time (Balint RF, Larrick JW. Gene 1993, 137:109-118).

Many mutagenesis and selection strategies have been used to provide a therapeutic candidate antibody having high affinity binding domains. Broadly applicable mutagenesis strategies target the CDRs or the whole V gene with high or low levels of randomization, respectively. When clones from (semi)synthetic libraries are used, CDRs not diversified in the primary library may be targeted. Residues that affect affinity may also be determined experimentally (e.g., by alanine, shotgun or homology scanning) or identified on the basis of structural knowledge and then randomized.

A successful strategy is to modify the CDR3 regions of the VH and VL or VH alone. Substitutions the central region of CDR3 or other CDR region can be done in combination with alterations of residues at the periphery, identified by a process of error-prone mutagenesis, such as by using a display-based selection and high-throughput sequencing. Mutations in the CDR loops provide new contacts or influence the positioning of side chains contacting the antigen or replace low-affinity contact residues with those with more favorable binding kinetics. Once the affinity is below 10-20 pM, it can be further increased through subtle structural changes in the binding site (e.g., through mutations in the framework regions of the variable regions that are not hypervariable and that modulate CDR loop flexibility or affect the orientation of the VH and VL domain.

Thus, in the process of developing a Mab as a therapeutic candidate, a parental antibody with the desired specificity of binding may be selected by, e.g. binding to the target in one or more formats, such as a purified protein in solid or liquid phase or as target antigen displayed on a cell surface. The parental Mab encoding sequence may be mutagenized or altered by a process of directed evolution or site directed mutagenesis or other process to produce sequences encoding variant antibodies with altered amino acid residues. These variants will constitute a limited library or family of related antibodies which may be further selected for enhanced affinity. The parental Mab or a variant thereof with the desired binding specificity and enhanced affinity may be further mutagenized to provide an altered set of variants with yet a different set of biophysical properties but derived from a parent antibody with the desired binding specificity and affinity. The method of the invention may be used to select among the limited library or family of structurally related Mabs for the species exhibiting the best potential for high concentration formulation or highest solubility. The method of the invention can be used to rank the potential for high concentration formulation among various related or unrelated antibodies which ranking may be among several success criteria for choosing a candidate Mab for commercial development.

When the initial screening is done using the VH and VL domains or Fab' (VH-CH1 and VL-CL) these fragments may be used to create a full length antibody of any class/subclass. The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see e.g., Kabat, E. A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region. For a Fab fragment heavy chain gene, the VH-encoding DNA can be operatively linked to another DNA molecule encoding only the heavy chain CH1 constant region. The light chain constant region can be a kappa or lambda constant region.

To express the antibodies, or antibody portions of the invention, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present).

## Chromatographic Materials, Methods, and Formats

Protein interactions using a protein immobilized on chromatographic particles and the retention of the same or a related protein measured in isocratic elution, can be performed using a variety of supports suitable for immobilization of proteins. The relative retention of the protein reflects the average protein interactions and is related to the second virial coefficient which, in turn, can be described as a function of the interaction range and sign (repulsive or attractive) which is unique for a particular protein structure and set of conditions.

For antibody interaction chromatography of the method of the invention, homogenous or heterogenous preparations of antibodies may be coupled to preactivated resin using procedures that generally involve reactions of an activated amino or carboxyl group-derivatized solid surface with amino-or thiol-containing biomolecules. After activation, or after introduction of a functionalized spacer, these groups offer direct attachment sites. Most of these functional groups (such as NHS esters, isothiocyanates, etc.) hydrolyze in an aqueous environment and become non-reactive (i.e., chemically inactive) after a short period thus providing a suitable medium for chromatographic studies. The use of amine and thiol group coupling chemistry can be achieved in solutions of pH 7-9. Numerous other chemistries are available for covalent coupling or antibodies to solid supports, including but not limited to activated epoxy and formyl resins. Such resins are commercially available under the names Toyopearl AF-Tresyl-650M, Toyopearl AF-Epoxy-650, Toyopearl AF-Carboxy-650, Toyopearl AF-Amino-650, and Toyopearl AF-Formyl-650 (Tosoh Biosciences), Hi-Trap NHS-activated column, CNBr-activated Sepharse, Activated Thiol Sepharose, Epoxy-activated Sepharose, EAH Sepharose, ECH Sepharose, and Activated-CH Sepharose (GE Healthcare), AminoLink, SulfoLink, CarboLink, CarboxyLink, PharmaLink, and UltraLink resins (Pierce).

Antibodies may also be linked to agarose resin using a hydrazide chemistry. It is also possible to immobilize antibodies to chromatographic supports using noncovalent forces, such as by binding to protein G or protein A affinity resins or by producing the antibody with a recognition or "tag" sequence that has affinity for a ligand affixed to the chromatography resin, such as but not limited to polyhistidine tags which bind $Ni^{2+}$ or $Co^{2+}$ charged resins. Other well known tags include LYTAG, c-myc, GST, FLAG, Maltose Binding Protein, Nus-tag, S-tag, Strep-tag, thioredoxin. Any of these means of immobilization of antibodies may be used in the method of the invention.

It will also be understood and recognized by those in the art that the incubation time, temperature, pH and protein concentration of the immobilization reaction mixture are the parameters for controlling surface immobilization when using chemical immobilization reactions.

Various chromatographic parameters need to be considered when performing AIC. In chromatographic systems used for SIC or for CIC, the nature of the interaction is related to the potential of mean force between protein molecules (Ahamed, et al. 2005 J Chromatography A 1089: 111-124). The interactive forces set up in a given column will be related to the amount of protein adsorbed per volume of resin, the concentration of protein in the mobile phase, and the amount of resin in terms of volume in so far as the changes in retention volume are due to antibody interactions only and thus are a measure of protein-protein interaction. More positive, or less negative values, for k' correspond to higher retention of the mobile protein molecules due to attractive interaction with stationary molecules. Similarly, more negative values of k' may also arise from repulsive interaction between stationary and mobile protein molecules. For the purpose of AIC, chromatography particles with wide pores may be desirable in order to minimize the mass transfer limitation and to ensure that immobilized protein molecules do not block the pore space for the mobile molecule to pass and interact outside the pore surface. In addition, the packed particles should have minimal interaction with mobile phase protein.

Tessier et al. (2002b, supra) found that the retention of protein depends on the injected concentration at higher surface coverage (33%), while the effect of injected concentration on retention volume is negligible at a surface coverage of 17-18%. At very high surface coverage, a free proteins molecule may have the opportunity to interact simultaneously with multiple immobilized molecules, which results in an injection concentration-dependent retention behavior. In addition, the higher surface coverage may block some particle pores, which hence become inaccessible to free proteins. Of course surface coverage will vary with the size and circumference of the protein immobilized thereto. In the method of the invention, a homogenous or heterogenous antibody preparation may be immobilization at from between 5 to 50 mg of protein per mL of column volume. In one embodiment of the practice of the method of the invention, densities of 5-30 mg of antibody per mL of resin for AIC studies are used. In one embodiment, the column has a total internal volume of 0.8 mL and the amount of antibody coupled to the resin is between 6.0 and 38 mg. Pressure is not important but typically the back pressures on the column is less than 1000 psi. However, other pressures may be operable.

Chromatographic resolution is also related proportionally to the volume to length ratio of the column. Columns with smaller volumes can be successfully employed if the flow rate and density of antibody coupling are adjusted.

Columns can be prepared using standard chromatography materials to include column volumes from 0.1 mL to 520 mL consisting of internal diameters of 3.0 mm to 2.6 cm and column lengths of 2.5 cm to 100 cm. The flow rate of chromatographic separation must also be considered. The faster the flow rate, the less time the solution and column bound antibodies are given to interact. Thus the slower the flow rate, the higher the resolution of separation. In the method of the invention, a flow rate of 0.1 mL/min can be used to provide adequate peak resolution with minimal experimental run time when the column volume is between 0.4 and 1.0 ml. In other instances the flow rate may be either faster or slower and may be between 0.01 and 10 mL/min.

In order to best predict solubility or other biophysical characteristics in a physiologically compatible or pharmaceutically acceptable solution, such as an aqueous medium, the compositions of the solutions used during the chromatographic testing of the antibody can be chosen from those known by those skilled in the art to be either physiologically compatible or pharmaceutically acceptable. In one embodiment, a buffered saline solution having a pH close to that found in one or more human body compartments is chosen, such as the commonly used PBS solution. In other embodiments, unbuffered solutions or solutions which are hyper- or hypo-osmotic may be selected for a particular application.

Another chromatographic consideration is the concentration of the antibody injected onto the column. Injection of high concentration solutions can lead to saturation of the column and thus minimal interactions between the injected and column bound antibodies. This degree of saturation varies with the density of the coupled material. Higher concentrations of injected materials also uses up greater quantities of antibodies for each run. Applicants have determined that the retention time of an antibody on columns is measurable when injecting an antibody over a range of concentrations from 0.1 to 10 mg/ml for column volumes of 0.8 mL and with a density of immobilized antibody of 10-30 mg/mL. A concentration of 0.1-0.2 mg/mL of antibody preparation with injection volumes of as little as 10 uL can be used routinely to provide an adequate signal in the UV detector while minimizing column saturation and reagent consumption. Thus, in the method of the invention the solubility of as little as 1 ug of antibody may be routinely estimated using the techniques disclosed herein.

**Nature of Immobilized Antibody**

For AIC, a substantially homogeneous antibody preparation may be immobilized on suitable chromatographic medium as described above. The antibody may be of the same species, class, and subclass as the test antibody or antibody variants to be screened. In one embodiment, the homogeneous antibody preparation is a parental antibody from which variants have been created for the purposes of improving affinity of the antibodies to a target antigen. In another embodiment, the test antibody preparation is of an antibody that is an affinity matured variant of a parental antibody, having enhanced target affinity as compared to a parental or prototype antibody, which is has been further substituted or modified.

In another embodiment, the immobilized antibody is a heterogenous mixture of antibodies from the same or different species as the test antibody and may represent multiple class and subclass antibodies with undefined target specificities. One example of such as mixture suitable for use in the method of the invention is a polyclonal human IgG isolated from human serum available for purchase from Sigma-Aldrich. IgG purified from bovine, goat, chicken, murine, porcine, rabbit, rat, and sheep sera are also commercially available from various sources. The purity of IgG samples is typically greater than 95%.

**Method of Calibrating the Column**

In order to rapidly screen a series of antibodies for solubility properties, applicants have determined a method of profiling highly soluble antibodies versus poorly soluble antibodies using representative "standards" of each type. Reference standards consist of antibodies for which the solubility in the mobile phase to be tested are known. It is preferable to have several standards that have differing solubilities (From 10 to >150 mg/mL) in the examined mobile phase. In order to generate a set of standards, solubility of proteins Mab67, Mab1, enteracept and pavalizumab were determined in PBS. These standards were then run over each AIC column generated to determine the approximate retention times of antibodies with the corresponding solubilities.

Having been described in general terms, the method of the invention will be further disclosed in the following examples.

**EXAMPLE 1: RELATIONSHIP BETWEEN ANTIBODY SOLUBILITY AND B22**

$B_{22}$ values for a human antibody of IgG1λ class, known as Mab67 having binding specificity for a human cytokine target antigen, derived from an Fab phage display library as described in WO2006124451A2, were determined in five potential formulation buffers in which solubility data was available for this antibody. Mab67 was diluted to a concentration of 3 mg/mL in 6 potential formulation buffers. The stock solutions were filtered using 0.22 μm Millex

Millipore filters. Protein concentrations were determined spectrophotometrically using $\varepsilon$ (A280 nm, 1 cm, 1 mg/mL) = 1.4. Static light scattering (SLS) was used for the determination of $B_{22}$. The instrument design consisted of a laser beam and a UV beam simultaneously delivered with a bifurcated fiber to a low volume (approximately 1 uL) flow cell. This arrangement allowed for simultaneous detection of scattered intensity (I) and protein concentration (c). A small volume of protein solution (approximately 20 uL) at 3 mg/mL in a defined buffer solution was injected into the flow stream, which consisted of a syringe pump, Rheodyne injector, membrane filters and the scattering cell. As the protein eluted through the scattering cell, data for scattering intensity and solution UV transmission were recorded. The experiment time for a single injection was approximately 40 minutes and several injections of the same sample were made to prove reproducibility. Debye plots (c/I vs. c) were made and the $B_{22}$ values were obtained from the slopes of said plots by fitting a linear line to the data.

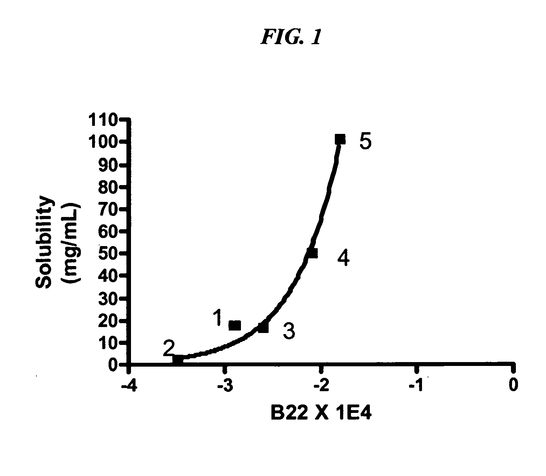

In order to determine the solubility of MAB67 in each of these formulation solutions, each antibody solution was added to Vivaspin ultrafiltration spin column with a molecular weight cut-off of 30,000 daltons (Vivascience Ltd.) at room temperature and centrifuged at speed 3,000 x g for 20 min. Once the volume was reduced to 2 ml, supernatants were transferred to a 2-ml spin column and centrifuged at speed 4,000 x g for 20 min. After sample volumes were reduced to 500 $\mu$l, samples were transferred to a Vivaspin 500 spin column and centrifuged at 15,000 x g for 15 min. This procedure was repeated until precipitation was observed or the sample volume was reduced to less than 10 $\mu$L. Supernatants were incubated at room temperature overnight to reach equilibrium. After spinning down the precipitation, the protein concentrations were determined by absorbance at 280 nm with appropriate dilution. If the concentration reached more than 100 mg/ml, the spin procedure was stopped and the protein concentration was determined as previously described, even though precipitation was not observed. Figure 1 shows that $B_{22}$ becomes more positive as the solubility of MAB67 increases. This data suggests that a $B_{22}$ value of greater than $-2 \times 10^{-4}$ is necessary to achieve the solubility required for subcutaneous administration of a therapeutic antibody (> 100 mg/mL).

$B_{22}$ measurements are characteristic for each protein and solution condition. Phosphate buffered saline (PBS) mimics physiologic pH and ionic strength. $B_{22}$ values for four test antibodies or antibody-derived proteins: MAB67, pavilizumab (SYNAGIS®), enteracept (ENBREL®, a polypeptide-Fc fusion protein), and mAb1. All were dialyzed extensively against PBS and the $B_{22}$ values were determined by static light scattering methods as described above. The solubility of each protein in PBS was determined by microfiltration as described as described above. The maximum concentrations of Mab1, pavilizumab, and enteracept were not determined due to limiting amounts of recombinant protein. No precipitation was observed at the concentrations listed and * indicates that maximum solubility was not determined due to limited quantities of sample. These values are shown in Table 1.

Table 1.

| Molecule | $B_{22}$ by SLS | Solubility in PBS | $t_r$ (min) | k' |
|---|---|---|---|---|
| MAB67 | -0.00071 | 13 mg/mL | 9.48 | 0.3695 |
| Mab1 | -0.00009 | >50 mg/mL* | 7.22 | 0.0435 |
| pavilizumab | -0.00003 | > 140 mg/mL* | 7.19 | 0.0390 |
| enteracept | 0.00000 | >87 mg/mL* | 6.93 | 0.0016 |

MAB67 was concentrated to only 13 mg/mL before precipitation was observed. The more soluble proteins have $B_{22}$ values > $-0.9 \times 10^{-4}$ while $B_{22}$ for MAB67 is $-7.1 \times 10^{-4}$. Thus, a $B_{22}$ value in PBS greater than approximately $-0.9 \times 10^{-4}$ was found to indicate solubility in the range which provides the potential for subcutaneous delivery for a therapeutic antibody candidate.

**EXAMPLE 2: USE OF ANTIBODY INTERACTION CHROMATOGRAPHY TO MEASURE A SECOND VIRIAL COEFFICIENT**

The methods used to determine the $B_{22}$ value of a protein generally require quantities of recombinant protein > 10 mg as well as time consuming steps of immobilizing the protein to a chromatography resin or the use of specialized equipment for light scattering methods. A method of calculating the $B_{22}$ value of a therapeutic protein is described that uses only 1 ug of the protein of interest. The method is based on idea that aggregation prone antibodies interact not only with self, but also with other antibodies. In other words, for antibodies, second virial cross coefficients ($B_{23}$) (Tessier, et al., Protein Sci 13: 1379-90, 2004) are good predictors of $B_{22}$ values. Thus, an antibody-coupled chromatography column can be calibrated by determining the retention time of various antibodies with known $B_{22}$ values on such a column.

In order to test this method, MAB67 was coupled to Toyopearl AF-tresyl-650M chromatography resin (Tosoh Biosciences, Montgomeryville, PA) by adding 10 mL of a 1 mg/mL solution of MAB67 in coupling buffer (0.1 M NaHCO3 pH 8.1, 0.5 M NaCl) to 1 mL of 0.25 g of dry resin swollen in 1 mL of coupling buffer. The reaction was allowed to proceed for 5 hours at room temperature before washing twice with 10 mL of coupling buffer. The remaining unreacted tresyl groups were capped by incubating the resin in 0.1 M Tris-HCl pH 8.1, 0.5 M NaCl overnight at 4°C. The coupled resin was packed in a 6.6 X 50 mm borosilicate chromatography column (Omnifit, Boonton, NJ) in PBS at a flow rate of 1 mL/min until the bed height stabilized. Final volume of the column was ~ 0.8 mL. The integrity of the packing procedure was verified by injecting a 10 $\mu$L sample of 10% acetone. A control column was generated by blocking 1 mL of swollen resin in 0.1 M Tris-HCl pH 8.1, 0.5 M NaCl overnight at 4°C without coupling any protein to the surface. This resin was then packed into an identical column as described above.

The retention time of the test proteins described above were determined on the MAB67 coupled column by diluting each antibody to a concentration of 0.1 mg/mL in PBS and injecting 10 $\mu$L onto the column at a flow rate of 0.1 mL/min using a mobile phase of PBS. The retention time of each sample was also determined on the control (non antibody coupled) column using a Beckman Coulter System Gold HPLC system equipped with an autosampler at room temperature. The column was equilibrated with at least 3 column volumes of PBS between runs. Peaks were visualized by monitoring absorbance at 214 nm and 280 nm using a UV detector. Retention times were determined using Karat32 software.

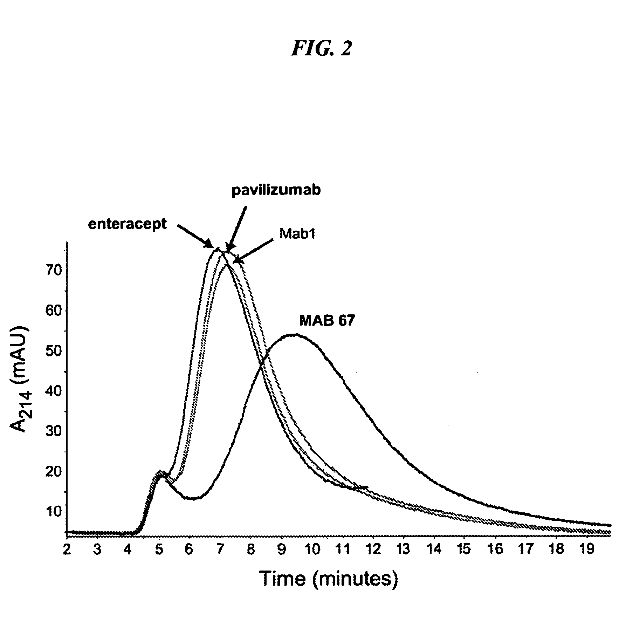

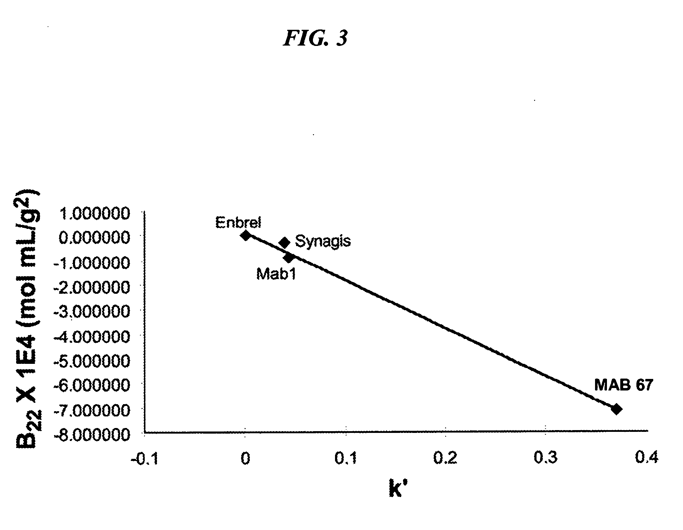

Figure 2 shows the chromatographic traces of these molecules demonstrating the differences in retention time experienced by each. MAB67 is more strongly retained on this column than the other proteins tested. The retention times for each sample were used to calculate the retention factor (k') using the formula as described (see Definitions section). These values were compared to the $B_{22}$ values established by SLS and solubility (Example 1, Table 1). Further, when $B_{22}$ values from Example 1 were plotted against k' (Figure 3) the linear correlation ($R^2$ = 0.99). This relationship demonstrates that an immobilized related protein, Mab column, can be used to accurately estimate the $B_{22}$ values of different antibodies and antibody-derived molecules.

## EXAMPLE 3: USE OF AIC TO SCREEN VARIANTS OF AN ANTIBODY FOR IMPROVED SOLUBILITY

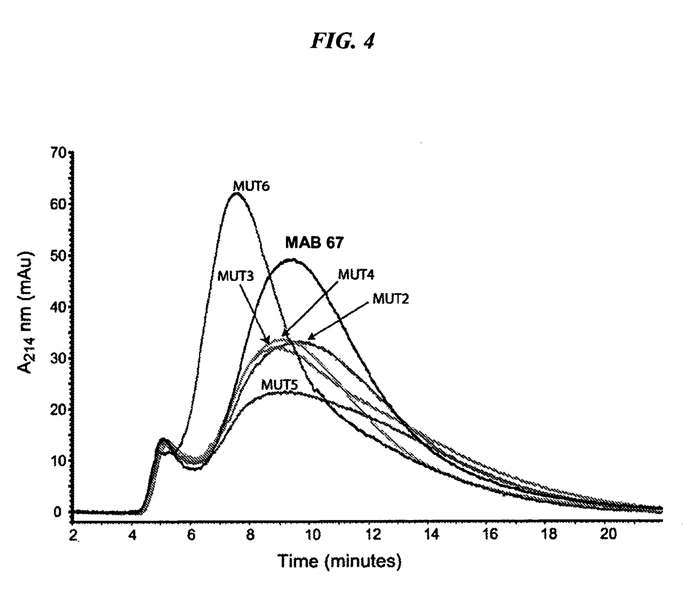

AIC using the MAB67 coupled column described above was used to screen a number of MAB67 variants designed to improve the solubility of this antibody by altering surface hydrophobicity and/or the isoelectric point. The retention times of MAB67 and of 5 variants, MUT2, MUT3, MUT4, MUT5, and MUT6 were measured on the MAB67 coupled column described in Example 2, by injecting 10 $\mu$L of a 0.1 mg/mL solution of each antibody in PBS over the column at a flow rate of 0.1 mL/min. Elution from the column was monitored by UV absorbance at 214 nm and 280 nm. PBS was used as the mobile phase for each antibody. Figure 4 shows the chromatographic traces of each antibody on the MAB67 column. Retention times of each antibody were determined from the corresponding trace and the k' calculated as described above. Measurements were made in triplicate with the exception of MUT4, which was only measured once. Table 2 summarizes the k' values (Mean and Std Dev) obtained for each antibody. With the exception of MUT6, all variants had k' values within experimental error of MAB67, which would indicate similarly poor solubility. Each antibody was concentrated in PBS by microfiltration as described in Example 1 in order to estimate solubility (Table 2), where * indicates that maximum solubility was not determined due to limited quantities of sample. While MAB67 and mutants MUT2, MUT3, MUT4, and MUT5 could only be concentrated to 13-25 mg/mL before precipitation was observed, MUT6 was concentrated to over 163 mg/mL without precipitating.

These data show that the calculated k' value can be used to distinguish differences in solubility of antibody variants. The solubility of MUT6, which was significantly enhanced compared to the starting material (MAB67), was predicted by the reduction in mean k' by almost 10-fold compared with the parental Mab. Moreover, k' also successfully predicted that the solubility of other variants were not substantially improved as shown by non-significant change in the k' from the parental Mab.

Table 2.

| Mab | $t_r$ | $t_m$ | k' | Solubility in PBS (mg/mL) |
|---|---|---|---|---|
| MAB67 | 10.4 | 6.7 | 0.56 $\pm$ 0.25 | 13 |
| MUT2 | 9.2 | 6.7 | 0.36 $\pm$ 0.04 | 25 |
| MUT3 | 8.9 | 6.7 | 0.32 $\pm$ 0.03 | 13 |
| MUT4 | 9.1 | 6.7 | 0.32 | 17 |
| MUT5 | 9.6 | 6.7 | 0.43 $\pm$ 0.11 | 12 |
| MUT6 | 7.1 | 6.7 | 0.06 $\pm$ 0.03 | > 163 * |

## EXAMPLE 4: RETENTION OF PROTEINS ON POLYCLONAL hIgG COLUMN

Our results suggest that an aggregation prone antibody, such as MAB67, interacts strongly not only with self, but also with other antibodies. In order to eliminate the need for producing recombinant protein to be immobilized on a column, as well as develop a more generalized procedure, polyclonal IgG isolated from human serum was coupled to an NHS-activated column. Purified IgG from human serum was obtained from Sigma (catalog No. 14506-100MG) and resuspended in 0.1 M NaHCO3 pH 8.1, 0.5 M NaCl at a concentration of 8.2 mg/mL. A prepacked HiTrap NHS-activated column (GE Healthcare # 17-0716-01) with a column volume of 1 mL was equilibrated with 5 mL of 1 mM HCl. 5 mL of the hIgG solution was then passed back and forth over the column at room temperature every 30 minutes for 5 hours using a syringe. Coupling efficiency was determined by measuring UV absorbance (280 nm) to determine the protein concentration left in solution. A resulting density of 29.5 mg of hIgG per 1 mL of column was achieved. After coupling, any remaining reactive groups were blocked by incubating the column overnight at 4°C in 0.1 M Tris-HCl pH 8.1, 0.5 M NaCl. The column was equilibrated in PBS prior to performing measurements.

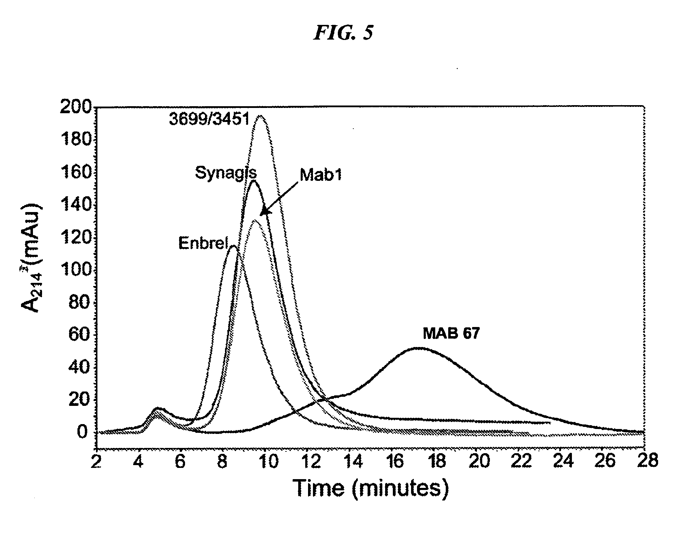

In order to measure the retention time of proteins on this column, 10 $\mu$L of 0.1 mg/mL solutions of MAB67, palivizumab, enteracept, and MUT6 in PBS were injected onto the column at a flow rate of 0.1 ml/min and a mobile phase of PBS and the retention time monitored by UV absorbance at 214 nm and 280 nm (Table 3). Figure 5 shows that MAB67 is retained significantly longer on this column than the more soluble proteins.

Table 3.

| Protein | $t_r$ (Minutes) | k' |
|---------|-----------------|-----|
| MAB67 | 17.4 | 0.802 |
| MUT6 | 9.8 | 0.086 |
| pavilizumab | 9.5 | 0.063 |
| enteracept | 8.5 | 0.040 |

The results obtained using this hIgG column are consistent with those obtained using AIC and light scattering methods (Example 1 and Example 2) and demonstrate that retention time and k' from a column coupled with polyclonal hIgG is predictive of the solubility of each antibody, as Mab67 has a k' value approximately 10-fold greater than the more soluble molecules.

## EXAMPLE 5: THE EFFECT OF RESIN TYPE, COUPLING DENSITY, FLOW RATE, INJECTION VOLUME, AND INSTRUMENT

In an effort to optimize the resolution and and control variability of the AIC method, several parameters were tested using polyclonal IgG isolated from human serum. These parameters are resin type, flow rate, injection volume, and coupling density. Two different HPLC systems were employed which gave similar results (Beckman System Gold LC (Beckman Coulter, Fullerton, CA) and the Ettan LC (GE Healthcare Bio Sciences, Piscataway, NJ)).

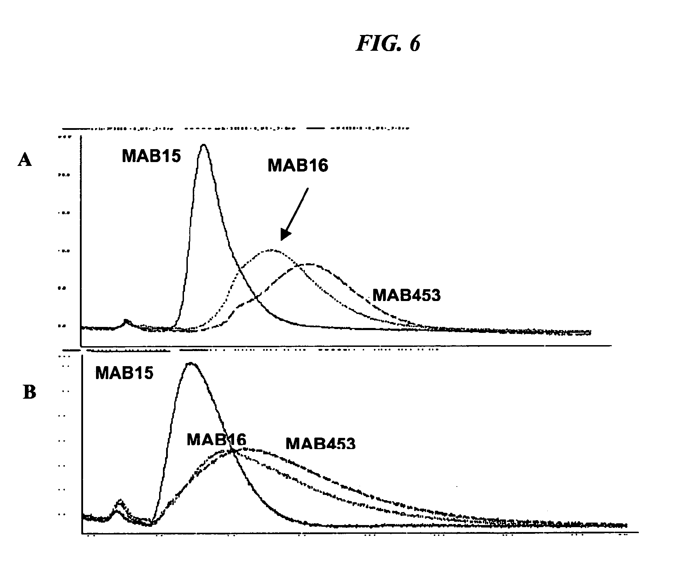

Two types of resins were tested: Toyopearl AF-Tresyl-650M and an NHS-activated Sepharose. The NHS resin is a HiTrap NHS activated pre-packed column whereas the Toyopearl AF-Tresyl resin has to be manually packed. Three antibodies MAB 15, MAB16, and MAB453 were used in this study. Figure 6 shows that the NHS-activated resin yields the best resolution.

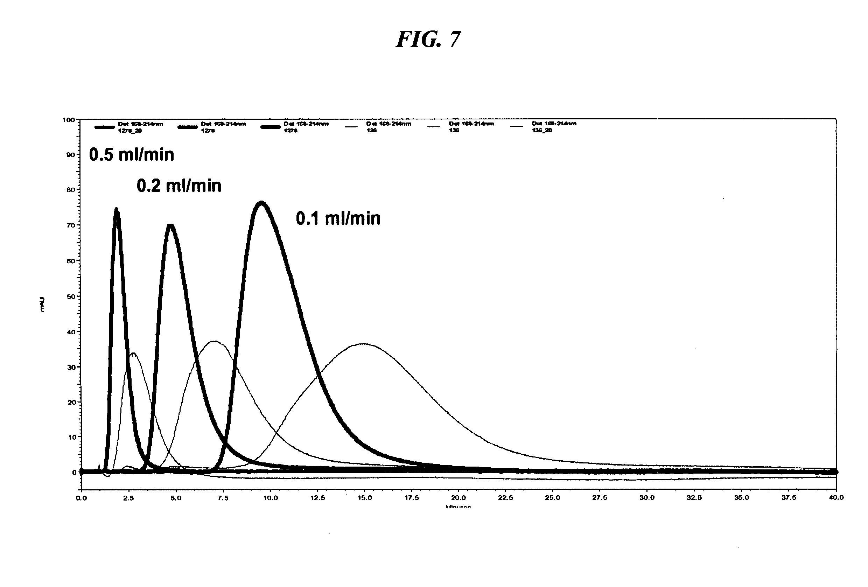

Three flow rates of 0.1 ml/min, 0.2 ml/min and 0.5 ml/min were tested at the coupling density of 30 mg/ml using MAB 15 and MAB16. The flow rate that provided best resolution among the antibodies was found to be 0.1 ml/min, the slowest flow rate (Fig. 7).

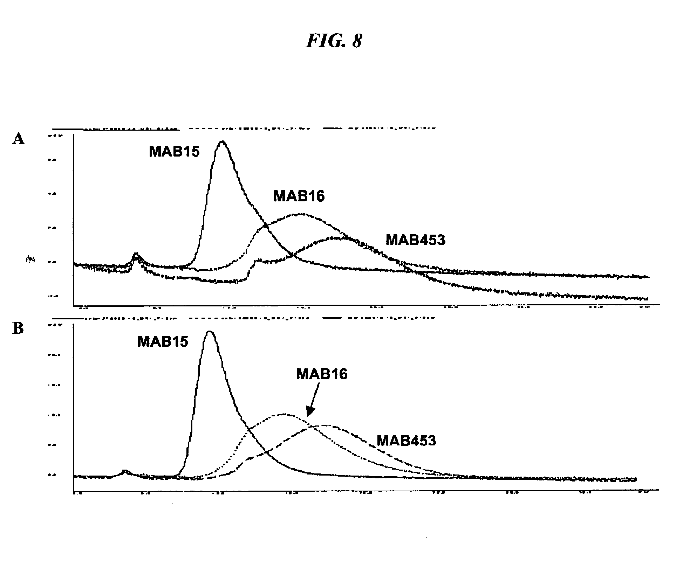

Injection volume of 10 ul and 20 ul were tested to find the lower limit that still yields reliable result. Three antibodies MAB15, MAB16, and MAB453 were used in this study. It was found that 20 ul which yields more consistent data as shown in Figures 8. The two HPLC systems used in the assessment were compared at the optimum flow rate and injection volume conditionswith similar results (Fig. 8). These method parameters were employed in the coupling density assessment.

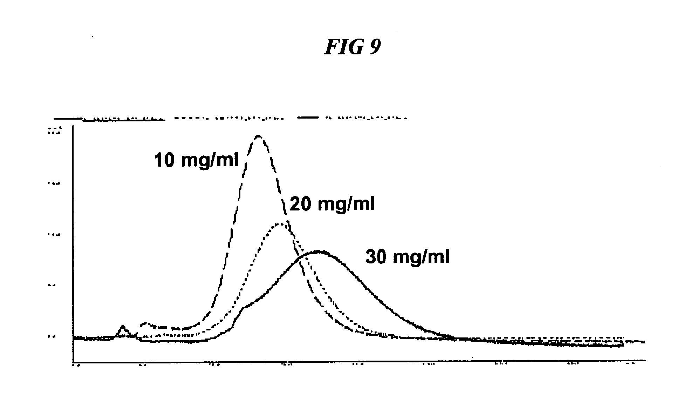

NHS Columns were packed at coupling densities of 30 mg/ml, 20 mg/ml and 10 mg/mlto test the effect of the coupling density. The flow rate of 0.1 ml/min and injection volume of 20 ul of 0.1 mg/ml MAB453, MAB15, MAB16, MAB67 solutions were used in this study. Figure 9 shows the effect of coupling density on MAB453 demonstrating that 30 mg/ml coupling density gave the best resolution.

**EXAMPLE 6: SOLUBILITY RANKING OF THERAPEUTIC ANTIBODY CANDIDATES ON HUMLAN IGG COLUMN**

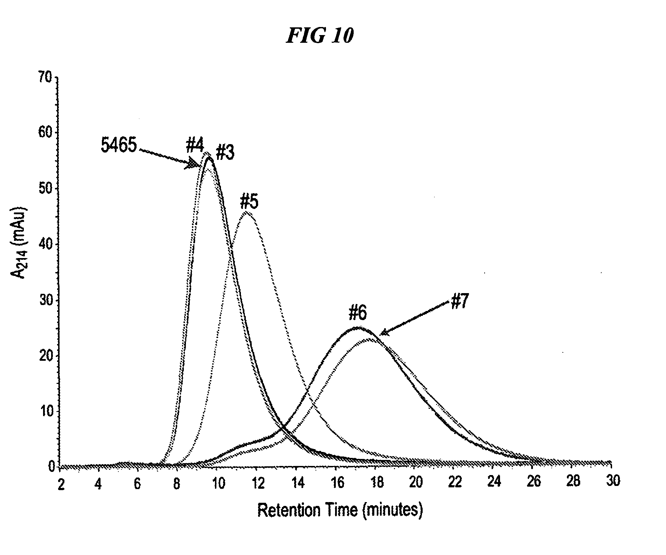

A panel of therapeutic antibody candidates were screened for interaction with column bound polyclonal human IgG in order to rank the solubility of these antibodies. A Fab phage display library in which heavy chain CDR3 and light chain CDR3 are diversified was screened for binding to a target protein of therapeutic of interest. After three rounds of selection from this library, six parental antibodies were isolated and converted to full Mabs by standard cloning methods, and expressed and purified from HEK293 cells. Four parent antibodies, designated 5409, 5465, 5415, and 5544 were selected for affinity maturation based on *in vitro* neutralization of the target antigen in biological assays. For affinity maturation, secondary phage display libraries were generated from these parent molecules in which sequence diversity was introduced into heavy chain CDR2 and light chain CDR1 and CDR3 for each parent. After three rounds of panning against the target antigen, 15 Fabs were selected for further analysis. Fabs were converted to full length Mabs by standard cloning methods and expressed and purified from HEK293 cells. Each candidate, along with the parental antibodies, were screened for interaction with polyclonal human IgG by injecting 10 $\mu$L of a 0.2 mg/mL solution of each in antibody PBS onto the hIgG column described in Example 4, at a flow rate of 0.1 ml/min and a mobile phase of PBS and the retention time monitored by UV absorbance at 214 nm and 280 nm. Figure 10 shows an example of the chromatographic traces obtained for antibodies 3, 4, 5, 6, and 7, all of which were derived from parent antibody 5465. Table 4 lists the retention time for each antibody tested on this column. Figure 10 shows that the antibodies screened have differences in the strength of interaction with polyclonal IgG as judged by the retention times on the IgG column. Antibodies 3, 4, and 5465 show less interaction ($t_r < 10$ minutes), while antibodies 6 and 7 show a greater degree interaction with the IgG as retention time has more than doubled with respect to 5465.

Similar data was generated for the antibodies derived from the other parental Mabs (Table 4). This data was used to rank the solubility of each antibody such that antibodies with a shorter retention time on the column are ranked as having a higher solubility potential where * indicates that maximum solubility was not determined due to limited quantities of sample. The $t_r$ and thus k' obtained on this column was tested as a predictor of protein solubility in PBS by determining the solubility of a number of the antibodies described in Table 4 by microfiltration as described above. Antibodies 6 and 7, which retained on the column the longest with k' values of 0.994 and 1.018, were only soluble to 10 and 9 mg/mL respectively. However, antibodies 9, 12, 14, and 15 all had k' values greater than 0.329 and could be concentrated to at least 108 mg/mL in PBS. Determination of the upper limit of solubility for these antibodies was not possible due to limiting amounts of recombinant protein. In order to pick a lead molecule, the solubility rankings obtained from this analysis was combined with other rankings of affinity for the target antigen, ability to neutralize the target antigen in *in vitro* assays, and ability to neutralize the cynomolgous antigen in *in vitro* assays.

The data presented in this example demonstrate that the k' values for a series of antibodies generated by affinity maturation processes is predictive of the relative solubilities of those antibodies. The solubility of antibodies 6 and 7 was significantly reduced as compared to other candidates, as predicted by a k' value at least 3-fold larger than the more soluble candidates 9, 12, 14, and 15. Antibodies having a k' value of less than 0.33 measured on a polyclonal IgG-conjugated column were soluble at least 100 mg/ml. Thus, k' can be used as a means to rank the solubility of each antibody candidate using only 1 ug of recombinant material and can thus be used as a criteria for selecting a lead candidate molecule.

Table 4.

| Sample | Parent | $t_r$ (minutes) | k' | Solubility (mg/mL) |
|---|---|---|---|---|
| 5544 | | 9.45 | 0.062 | |
| 1 | 5544 | 10.58 | 0.179 | |
| 5409 | | 9.55 | 0.053 | |
| 2 | 5409 | 9.99 | 0.075 | |
| 5465 | | 9.62 | 0.102 | |
| 3 | 5465 | 9.68 | 0.114 | |
| 4 | 5465 | 9.58 | 0.099 | |
| 5 | 5465 | 11.53 | 0.334 | |
| 6 | 5465 | 17.15 | 0.994 | 10.0 |
| 7 | 5465 | 17.74 | 1.018 | 9.0 |
| 5415 | | 10.50 | 0.198 | |

EP 2 243 031 B1

(continued)

| Sample | Parent | $t_r$ (minutes) | k' | Solubility (mg/mL) |
|--------|--------|-----------------|-------|--------------------|
| 8 | 5415 | 10.76 | 0.209 | |
| 9 | 5415 | 10.54 | 0.209 | >108* |
| 10 | 5415 | 11.58 | 0.317 | |
| 11 | 5415 | 12.05 | 0.384 | |
| 12 | 5415 | 10.95 | 0.259 | >124* |
| 5544 | | 9.45 | 0.062 | * |
| 13 | 5544 | 12.78 | 0.421 | * |
| 14 | 5544 | 11.86 | 0.329 | >109* |
| 15 | 5544 | 11.12 | 0.217 | >119* |

## EXAMPLE 7: SCREENING OF HUMANIZED ANTIBODIES ON POLYCLONAL HUMAN IgG COLUMN

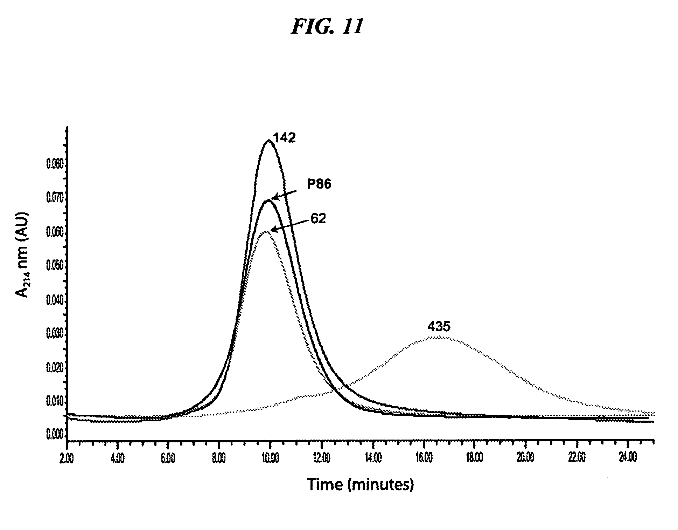

A murine anti-cytokine antibody (P86) was used to produce a collection of CDR-grafted variants with various fully human sequences of both the heavy and light chains. These humanized antibodies were tested for binding to the cytokine by ELISA and Biacore and assayed for neutralization of the cytokine in *in vitro* cell-based assays. Several humanized antibody sequences that retained binding and neutralization activity; 142, 62, and 453, were screened by interaction with a polyclonal human IgG column (Example 4) by injecting 10 μL of a 0.2 mg/mL solution of each antibody onto the column at a flow rate of 0.1 mL/min and monitoring elution by UV absorbance at 214 nm and 280 nm. PBS was used as the mobile phase. The chromatographic profiles of these humanized antibodies, as well as that of the parent antibody P86 are shown in Figure 11. MAB142 and MAB62 eluted at retention times within 0.1 minutes from that of the parent molecule (Table 5). MAB453, however, showed a markedly longer retention time on the hIgG column (retention time 16.7 mL) indicating potentially poor solubility.

Table 5.

| Antibody | $t_r$ (minutes) | k' | Solubility (mg/mL) |
|----------|-----------------|-------|--------------------|
| P86 | 9.9 | 0.098 | |
| MAB62 | 9.8 | 0.086 | >131* |
| MAB142 | 10.0 | 0.109 | >106* |
| MAB453 | 16.7 | 0.851 | 70.0 |

This retention is predictive of solubility as MAB62 and MAB142 could be concentrated over 106 mg/mL while MAB453 reached a maximal concentration in PBS of 70 mg/mL. This data demonstrates that k' values can be used to predict and rank the solubilities of a series of antibodies generated by humanization processes, as MAB453 has a k' value 8-fold greater than the others and was unable to be concentrated above 70 mg/mL in PBS. Moreover, k' predicted that candidates MAB62 and MAB142 are soluble in PBS to over 100 mg/mL concentration and also have k' values < 0.33 as shown in Example 6 for other sets of antibodies using the same column.

## REFERENCES

1. Balint RF, Larrick JW: Antibody engineering by parsimonious mutagenesis. Gene 1993, 137:109-118.

2. Glaser SM, Yelton DE, Huse WD: 1992. Antibody engineering by codon-based mutagenesis in a filamentous phage system. J Immuno 149:3903-3913.

3. Mossner, E., Koch, H. & Pluckthun, A. 2001. Fast selection of antibodies without antigen purification: adaptation of the protein fragment complementation assay to select antigen-antibody pairs J. Mol. Biol. 308, 115-122.

4. Urban, J.H. et al. 2005. Selection of functional human antibodies from retroviral display libraries. Nucleic Acids Res. 33, e35).

5. Odegrip, R. et al. 2004. CIS display: In vitro selection of peptides from libraries of protein-DNA complexes. Proc. Natl. Acad. Sci. USA 101, 2806-2810;

6. Reiersen, H. et al. 2005. Covalent antibody display-an in vitro antibody-DNA library selection system. Nucleic Acids Res. 33, e10).

7. Sepp, A., Tawfik, D.S. & Griffiths, A.D. 2002. Microbead display by in vitro compartmentalisation: selection for binding using flow cytometry. FEBS Lett. 532, 455-458.

8. George, A., and W. W. Wilson. 1994. Predicting protein crystallization from a dilute solution property. Acta Crystallog. D Biol. Crystallog. D50:361-365.

9. Tessier, P. M., A. M. Lenhoff, and S. I. Sandler. 2002. Rapid measurement of protein osmotic second virial coefficients by self-interaction chromatography. Biophys. J. 82:1620-1631.

10. Tessier, PM. Sandler, SI, Lenhoff, AM. 2004. Direct measurement of protein osmotic second virial cross coefficients by cross-interaction chromatography. J Protein Sci. 13: 1379-1390.

11. Valente, J.J., Payne, R.W., Manning, M.C., Wilson, W.W., Henry, C.S. 2005 Colloidal behavior of proteins: Effects of the second virial coefficient on solubility, crystallization and aggregation of proteins in aqueous solution. Curr Pharmaceutical Biotechnology 6(6);427-436, 2005.

12. Patro S.Y., Przybycien T.M. 1996. Self-interaction chromatography: A tool for the study of protein- protein interactions in bioprocessing environments. Biotechnol Bioengineer 52 (2):193-203.

13. Ruppert S., Sandler S.I., Lenhoff A.M. (2001) Correlation between the osmotic second virial coefficient and the solubility of proteins. Biotechnology Progress, 17 (1: 182-187.

14. Ho, J.G.S., Middelberg, A.P.J., Ramage, P., Kocher, H.P. (2003) The likelihood of aggregation during protein renaturation can be assessed using the second virial coefficient Protein Science, 12 (4):708-716.

15. T. Ahamed, B. N. A. Esteban, M. Ottens, G. W. K. van Dedem, L. A. M. van der Wielen, M. A. T. Bisschops, A. Lee, C. Pham, and J. Thommes. July 15, 2007. Phase Behavior of an Intact Monoclonal Antibody. Biophys. J. 93 (2): 610-619.

16. U.S. Patent No. 6974678 Henry, Wilson, & Garcia. Microfluidic device for measuring second viral coefficients (filed Oct. 8, 2002).

17. WO2006/131013 Esbatech AG (Swiss) humanized scFv or Fab See pp 53-56 & Table II (P. 56).

18. WO2006122191 DeLucas, Wilson, Lewis, & Bar. Improved microfluidic device.

19. Urban, J.H. et al. Selection of functional human antibodies from retroviral display libraries. Nucleic Acids Res. 33, e35 (2005).

20. Odegrip, R. et al. CIS display: In vitro selection of peptides from libraries of protein-DNA complexes. Proc. Natl. Acad. Sci. USA 101, 2806-2810 (2004).

21. Reiersen, H. et al. Covalent antibody display-an in vitro antibody-DNA library selection system. Nucleic Acids Res. 33, e10 (2005).

22. Sepp, A., Tawfik, D.S. & Griffiths, A.D. Microbead display by in vitro compartmentalisation: selection for binding using flow cytometry. FEBS Lett. 532, 455-458 (2002).

23. Mossner, E., Koch, H. & Pluckthun, A. Fast selection of antibodies without antigen purification: adaptation of the protein fragment complementation assay to select antigen-antibody pairs. J. Mol. Biol. 308, 115-122.

**Claims**

1. A method of measuring the solubility of an antibody of the IgG class having binding specificity for a target antigen in an pharmaceutically acceptable formulation by measuring the interaction between a first antibody and a second antibody, the method comprising:

   a) flowing a solution comprising a substantially homogeneous solution of the first antibody over a solid support contained in a defined column volume, wherein the second antibody is immobilized on a surface of the solid support; and
   b) measuring the time (retention time, tr) required for an amount of the solution comprising the first antibody to exit said column volume, wherein the retention time (tr) is a measure of interaction between the first antibody and the second antibody which correlates with the solubility of the first antibody in a pharmaceutically acceptable formulation,

   wherein the first and second antibody are different antibodies.

2. The method of claim 1, further comprising:

   determining the non-interaction time (tm) as the time required for the first antibody to exit the column by determining the time to pass through an underivatized column volume for said first antibody; and
   calculating a retention factor (k') from tr and tm, wherein $k' = t_r - t_m / t_m$ .

3. The method of claim 2, further comprising:

   comparing k' measured for a test antibody with the k' for an insoluble antibody flowed over the same column, where a smaller value of k' indicates a more soluble antibody.

4. The method of claim 2, further comprising:

   comparing k' measured for a test antibody with the k' for a highly soluble antibody flowed over the same column, where a similar value of k' indicates a more soluble antibody.

5. The method of claim 1, wherein the second antibody is a heterologous antibody preparation.

6. The method of claim 1, wherein the first antibody is a therapeutic candidate and the second antibody is an antibody with an undesirable susceptibility to aggregation.

7. The method of claim 1, wherein the space comprising the solid support has an internal volume of between 0.1 and 1.0 ml.

8. The method of claim 1, wherein the second antibody is a parental antibody and the first antibody is a variant thereof.

9. A method of ranking the solubility of an antibody of the IgG class having binding specificity for a target antigen in an pharmaceutically acceptable formulation by measuring the interaction between a first antibody and a second antibody comprising the method of claim 1, and wherein the method further comprises:

   c) repeating the method with other antibody candidates and using the tr values to rank the solubility of the antibody candidates having binding specificity to a target antigen.

**Patentansprüche**

1. Verfahren zur Messung der Löslichkeit eines Antikörpers der IgG-Klasse mit Bindungsspezifität für ein Zielantigen in einer pharmazeutisch unbedenklichen Formulierung durch Messen der Wechselwirkung zwischen einem ersten Antikörper und einem zweiten Antikörper, wobei man bei dem Verfahren

a) eine eine weitgehend homogene Lösung des ersten Antikörpers umfassende Lösung über einen in einem definierten Säulenvolumen enthaltenen festen Träger fließen lässt, wobei der zweite Antikörper auf einer Oberfläche des festen Trägers immobilisiert ist, und

b) die für das Austreten einer Menge der den ersten Antikörper umfassenden Lösung aus dem Säulenvolumen benötigte Zeit (Retentionszeit, tr) misst, wobei die Retentionszeit (tr) ein Maß für die Wechselwirkung zwischen dem ersten Antikörper und dem zweiten Antikörper darstellt, das mit der Löslichkeit des ersten Antikörpers in einer pharmazeutisch unbedenklichen Formulierung korreliert,

wobei es sich bei dem ersten und dem zweiten Antikörper um unterschiedliche Antikörper handelt.

2. Verfahren nach Anspruch 1, bei dem man ferner die Nichtwechselwirkungszeit (tm) als die für das Austreten des ersten Antikörpers aus der Säule benötigte Zeit bestimmt, indem man für den ersten Antikörper dessen Durchlaufzeit durch ein underivatisiertes Säulenvolumen bestimmt, und einen Retentionsfaktor (k') aus tr und tm berechnet, wobei gilt: k'= tr-tm/tm.

3. Verfahren nach Anspruch 2, bei dem man ferner für einen Testantikörper gemessenen k' mit dem k' für einen über die gleiche Säule geflossenen unlöslichen Antikörper vergleicht, wobei ein kleinerer Wert für k' einen löslicheren Antikörper anzeigt.

4. Verfahren nach Anspruch 2, bei dem man ferner für einen Testantikörper gemessenen k' mit dem k' für einen über die gleiche Säule geflossenen hochlöslichen Antikörper vergleicht, wobei ein ähnlicher Wert für k' einen löslicheren Antikörper anzeigt.

5. Verfahren nach Anspruch 1, wobei es sich bei dem zweiten Antikörper um eine heterologe Antikörperpräparation handelt.

6. Verfahren nach Anspruch 1, wobei es sich bei dem ersten Antikörper um einen therapeutischen Kandidaten und bei dem zweiten Antikörper um einen Antikörper mit einer unerwünschten Aggregationsneigung handelt.

7. Verfahren nach Anspruch 1, wobei der den festen Träger umfassende Raum ein Innenvolumen zwischen 0,1 und 1,0 ml aufweist.

8. Verfahren nach Anspruch 1, wobei es sich bei dem zweiten Antikörper um einen Elternantikörper und bei dem ersten Antikörper um eine Variante davon handelt.

9. Verfahren zur Einstufung der Löslichkeit eines Antikörpers der IgG-Klasse mit Bindungsspezifität für ein Zielantigen in einer pharmazeutisch unbedenklichen Formulierung durch Messen der Wechselwirkung zwischen einem ersten Antikörper und einem zweiten Antikörper, umfassend das Verfahren nach Anspruch 1, und wobei man bei dem Verfahren ferner

c) das Verfahren mit anderen Antikörperkandidaten wiederholt und die tr-Werte zur Einstufung der Löslichkeit der Antikörperkandidaten mit Hindungsspezifität für ein Zielantigen verwendet.

## Revendications

1. Procédé pour la mesure de la solubilité d'un anticorps de la classe des IgG, ayant une spécificité de liaison pour un antigène cible dans une formulation pharmaceutiquement acceptable, par mesure de l'interaction entre un premier anticorps et un deuxième anticorps, le procédé comprenant :

a) l'écoulement d'une solution comprenant une solution sensiblement homogène du premier anticorps sur un support solide contenu dans un volume de colonne défini, le deuxième anticorps étant immobilisé sur une surface du support solide ; et

b) la mesure du temps (temps de rétention, tr) nécessaire pour qu'une certaine quantité de la solution comprenant le premier anticorps sorte dudit volume de colonne, le temps de rétention (tr) étant une mesure de l'interaction entre le premier anticorps et le deuxième anticorps, qui présente une corrélation avec la solubilité du premier anticorps dans une formulation pharmaceutiquement acceptable,

le premier et le deuxième anticorps étant des anticorps différents.

2. Procédé de la revendication 1, comprenant :

la détermination du temps de non-interaction (tm) en tant que temps nécessaire pour que le premier anticorps sorte de la colonne, par détermination du temps pour que ledit premier anticorps traverse un volume de colonne non dérivatisé ; et le calcul d'un facteur de rétention (k') à partir de tr et de tm, avec k' = tr-tm/tm.

3. Procédé de la revendication 2, comprenant en outre :

la comparaison du k' mesuré pour un anticorps d'essai au k' pour un anticorps insoluble qui s'écoule sur la même colonne, une valeur de k' plus petite étant le signe d'un anticorps plus soluble.

4. Procédé de la revendication 2, comprenant en outre :

la comparaison du k' mesuré pour un anticorps d'essai au k' pour un anticorps très soluble qui s'écoule sur la même colonne, une valeur semblable de k' étant le signe d'un anticorps plus soluble.

5. Procédé de la revendication 1, dans lequel le deuxième anticorps est une préparation d'anticorps hétérologue.

6. Procédé de la revendication 1, dans lequel le premier anticorps est un candidat thérapeutique, et le deuxième anticorps est un anticorps ayant une sensibilité indésirable à l'agrégation.

7. Procédé de la revendication 1, dans lequel l'espace comprenant le support solide a un volume interne compris entre 0,1 et 1,0 ml.

8. Procédé de la revendication 1, dans lequel le deuxième anticorps est un anticorps parental, et le premier anticorps est un variant de ce dernier.

9. Procédé pour ranger la solubilité d'un anticorps de la classe des IgG ayant une spécificité de liaison pour un antigène cible dans une formulation pharmaceutiquement acceptable, par mesure de l'interaction entre un premier anticorps et un deuxième anticorps, comprenant le procédé de la revendication 1, le procédé comprenant en outre :

c) la répétition du procédé avec d'autres anticorps candidats, et l'utilisation des valeurs de tr pour ranger la solubilité des anticorps candidats ayant une spécificité de liaison à un antigène cible.

*FIG. 1*

## FIG. 2

*FIG. 3*

## FIG. 4

FIG. 5

EP 2 243 031 B1

*FIG. 6*

*FIG. 7*

EP 2 243 031 B1

FIG. 8

**FIG 9**

## FIG 10

## FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 05080422 A
- WO 2006131013 A
- EP 0929578 A
- WO 9306213 A
- WO 9201047 A
- WO 2006124451 A2
- US 6974678 B, Henry, Wilson, & Garcia
- WO 2006122191 A, DeLucas, Wilson, Lewis, & Bar

### Non-patent literature cited in the description

- **WARD et al.** *Nature,* 1989, vol. 341, 544-546
- **BIRD et al.** *Science,* 1988, vol. 242, 423-426
- **HUSTON et al.** *Proc. Natl. Acad Sci. USA,* 1988, vol. 85, 5879-5883
- **DEMORUELLE et al.** *Acta Crystallogr D Biol Crystallogr,* 2002, vol. 58, 1544-8
- **GEORGE ; WILSON.** *Acta Crystallogr D Biol Crystallogr,* 1994, vol. 50, 361-5
- **GUO et al.** *Journal of Crystal Growth,* 1999, vol. 196, 424-433
- **GARCIA et al.** *Biotechnol Prog,* 2003, vol. 19, 575-9
- **TESSIER et al.** *Acta Crystallogr D,* 2002, vol. 58, 1531-5
- **TESSIER et al.** *Biophys J,* 2002, vol. 82, 1620-31
- **TESSIER et al.** *Protein Sci,* 2004, vol. 13, 1379-1390
- **NEAL et al.** *Journal of Crystal Growth,* 1999, vol. 196, 377-387
- **VALENTE et al.** *Curr Pharm Biotechnol,* 2005, vol. 6, 427-36
- **BONDOS et al.** *Current Analytical Chemistry,* 2006, vol. 2 (2), 157-170
- **HAAS et al.** *J Phys Chem B.,* 1999, vol. 103, 2808-2811
- **KOHLER ; MILSTEIN.** *Nature,* 1975, vol. 256, 495
- Immunoglobulin genes. **TOMLINSON IM.** Encyclopedia of Immunology. Academic Press, 1998
- **VAUGHAN.** *Nature Biotechnology,* 1996, vol. 14, 309-314
- **SHEETS et al.** *PITAS (USA,* 1998, vol. 95, 6157-6162
- **HOOGENBOOM ; WINTER.** *J. Mol. Biol.,* 1991, vol. 227, 381
- **MARKS et al.** *J. Mol. Biol.,* 1991, vol. 222, 581
- **MCCAFFERTY et al.** *Nature,* 1990, vol. 348, 552-554
- **GRIFFITHS et al.** *EMBO J.,* 1993, vol. 12, 725-734
- **URBAN, J.H. et al.** *Nucleic Acids Res.,* 2005, vol. 33, e35
- **ODEGRIP, R et al.** *Proc. Natl. Acad. Sci. USA,* 2004, vol. 101, 2806-2810
- **REIERSEN, H. et al.** *Nucleic Acids Res.,* 2005, vol. 33, e10
- **SEPP, A. ; TAWFIK, D.S. ; GRIFFITHS, A.D.** *FEBS Lett.,* 2002, vol. 532, 455-458
- **MOSSNER, E. ; KOCH, H. ; PLUCKTHUN, A.** *J. Mol. Biol.,* 2001, vol. 308, 115-122
- **GLASER SM ; YELTON DE ; HUSE WD.** *J Immuno,* 1992, vol. 149, 3903-3913
- **BALINT RF ; LARRICK JW.** *Gene,* 1993, vol. 137, 109-118
- **KABAT, E. A. et al.** Sequences of Proteins of Immunological Interest. U.S. Department of Health and Human Services, 1991
- **AHAMED et al.** *J Chromatography A,* 2005, vol. 1089, 111-124
- **TESSIER et al.** *Protein Sci,* 2004, vol. 13, 1379-90
- **BALINT RF ; LARRICK JW.** Antibody engineering by parsimonious mutagenesis. *Gene,* 1993, vol. 137, 109-118
- **GLASER SM ; YELTON DE ; HUSE WD.** Antibody engineering by codon-based mutagenesis in a filamentous phage system. *J Immuno,* 1992, vol. 149, 3903-3913
- **MOSSNER, E. ; KOCH, H. ; PLUCKTHUN, A.** Fast selection of antibodies without antigen purification: adaptation of the protein fragment complementation assay to select antigen-antibody pairs. *J. Mol. Biol.,* 2001, vol. 308, 115-122
- **URBAN, J.H. et al.** Selection of functional human antibodies from retroviral display libraries. *Nucleic Acids Res.,* 2005, vol. 33, e35

- **ODEGRIP, R. et al.** CIS display: In vitro selection of peptides from libraries of protein-DNA complexes. *Proc. Natl. Acad. Sci. USA,* 2004, vol. 101, 2806-2810
- **REIERSEN, H. et al.** Covalent antibody display-an in vitro antibody-DNA library selection system. *Nucleic Acids Res.,* 2005, vol. 33, e10
- **SEPP, A. ; TAWFIK, D.S. ; GRIFFITHS, A.D.** Microbead display by in vitro compartmentalisation: selection for binding using flow cytometry. *FEBS Lett.,* 2002, vol. 532, 455-458
- **GEORGE, A. ; W. W. WILSON.** Predicting protein crystallization from a dilute solution property. *Acta Crystallog. D Biol. Crystallog.,* 1994, vol. D50, 361-365
- **TESSIER, P. M. ; A. M. LENHOFF ; S. I. SANDLER.** Rapid measurement of protein osmotic second virial coefficients by self-interaction chromatography. *Biophys. J.,* 2002, vol. 82, 1620-1631
- **TESSIER, PM ; SANDLER, SI ; LENHOFF, AM.** Direct measurement of protein osmotic second virial cross coefficients by cross-interaction chromatography. *J Protein Sci.,* 2004, vol. 13, 1379-1390
- **VALENTE, J.J. ; PAYNE, R.W. ; MANNING, M.C. ; WILSON, W.W. ; HENRY, C.S.** Colloidal behavior of proteins: Effects of the second virial coefficient on solubility, crystallization and aggregation of proteins in aqueous solution. *Curr Pharmaceutical Biotechnology,* 2005, vol. 6 (6), 427-436

- **PATRO S.Y. ; PRZYBYCIEN T.M.** Self-interaction chromatography: A tool for the study of protein- protein interactions in bioprocessing environments. *Biotechnol Bioengineer,* 1996, vol. 52 (2), 193-203
- **RUPPERT S. ; SANDLER S.I. ; LENHOFF A.M.** Correlation between the osmotic second virial coefficient and the solubility of proteins. *Biotechnology Progress,* 2001, vol. 17 (1), 182-187
- **HO, J.G.S. ; MIDDELBERG, A.P.J. ; RAMAGE, P. ; KOCHER, H.P.** The likelihood of aggregation during protein renaturation can be assessed using the second virial coefficient. *Protein Science,* 2003, vol. 12 (4), 708-716
- **T. AHAMED ; B. N. A. ESTEBAN ; M. OTTENS ; G. W. K. VAN DEDEM ; L. A. M. VAN DER WIELEN ; M. A. T. BISSCHOPS ; A. LEE ; C. PHAM ; J. THOMMES.** Phase Behavior of an Intact Monoclonal Antibody. *Biophys. J.,* 15 July 2007, vol. 93 (2), 610-619
- **MOSSNER, E. ; KOCH, H. ; PLUCKTHUN, A.** Fast selection of antibodies without antigen purification: adaptation of the protein fragment complementation assay to select antigen-antibody pairs. *J. Mol. Biol.,* vol. 308, 115-122